# FASCICULE DE BREVET EUROPEEN

(11) **EP 0 618 782 B1**
(45) Date de publication et mention de la délivrance du brevet: **16.10.1996**
(21) Numéro de dépôt: 93902344.6
(22) Date de dépôt: 17.12.1992
(51) Int. Cl.: A61B 6/04

(54) **LIT REGLABLE EN HAUTEUR**
HÖHENVERSTELLBARE PATIENTENLIEGE
HEIGHT ADJUSTABLE BED

(30) Priorité: 17.12.1991 FR 9115658
(43) Date de publication de la demande: 12.10.1994
(73) Titulaire: SOPHA MEDICAL, F-75008 Paris (FR)
(72) Inventeur: FLEURY, Christophe, F-78280 Guyancourt (FR); PARE, Christian, F-78370 Plaisir (FR)
(74) Mandataire: Schmit, Christian Norbert Marie
(86) Numéro de dépôt international: FR9201199
(87) Numéro de publication internationale: WO9311705

(56) Documents cités:
- EP-A- 0 283 083
- US-A- 4 912 754
- US-A- 5 048 069
- US-A- 5 067 145

## Description

La présente invention a pour objet un lit d'examen réglable en hauteur, utilisé à des fins médicales et notamment pour une tomographie par scintigraphie. Elle a pour objet de permettre un déplacement du lit de grande amplitude et selon la verticale. Elle permet au lit de se situer, en position maximale, à une hauteur par rapport au sol plus de deux fois supérieure à celle de sa position basse.

Actuellement, pour des tomographies par scintigraphie d'organes du corps humain, on allonge un patient à analyser sur un lit d'examen, en plaçant l'organe à examiner en face d'un détecteur d'une gamma caméra. L'examen tomographique consiste à acquérir une image en projection de l'organe par angle de vue, pour un grand nombre d'angles de vue. Ces angles de vue sont régulièrement espacés sur un secteur angulaire d'au moins 180°. Ils sont obtenus en faisant pivoter le détecteur de la gamma caméra autour du patient. On sait ensuite, avec des algorithmes de calcul, reconstituer, à partir des images en projection, l'image du volume examiné.

Dans une demande de brevet français n° 91 06962 déposée le 07 juin 1991 et publiée le 11 décembre 1992, on utilise, pour des tomographies d'organes du corps humain, une gamma caméra à deux têtes détectrices et dont le point de visée des têtes détectrices est décalé par rapport à l'axe de rotation de la gamma caméra. A cette fin, on effectue des angulations symétriques des têtes détectrices par rapport au plan horizontal. Ceci implique que les champs de détection des deux têtes détectrices ne sont plus parallèles. Puis, on fait pivoter la gamma caméra et, par conséquent, les têtes détectrices en conservant, lors de la rotation, comme point de visée un même lieu dans le corps humain.

Mais, du fait que le point de visée est décalé par rapport à l'axe de rotation de la gamma caméra, on est obligé d'animer la gamma caméra d'un mouvement de translation latérale tandis que le lit est animé d'un mouvement ascensionnel.

Un problème sérieux se pose néanmoins car, il faut prévoir, dans ce cas, un lit dont la position basse se situe à cinquante cinq centimètres au dessus du sol, dont la position haute se situe à environ cent vingt centimètres, et dont le déplacement s'effectue selon la verticale. Or, actuellement, les lits, généralement utilisés dans les hôpitaux, sont des lits dont le piétement est en forme de X. Ces lits présentent un premier inconvénient qui est celui de s'élever non pas verticalement mais en décrivant un arc de cercle. Un deuxième inconvénient provient de ce que le piétement en X laisse un dégagement qui n'est pas toujours le même sous le lit selon que le lit est en position haute ou basse. Hors cette solution, il n'y a pas de possibilité d'avoir des lits d'examen dont le débattement d'élévation soit d'un facteur deux ou même plus. Par ailleurs, compte tenu des poids impliqués (de l'ordre d'une tonne en tenant compte des coefficients de sécurité) toute structure envisageable est trop lourde ou trop fragile.

Un mécanisme de déplacement à chariot intermédiaire entraîné par une poulie et une chaîne est décrit par exemple pour le déplacement d'un tube à rayons X dans US-A-5 067 145.

Pour résoudre ces problèmes, dans la présente invention, on a eu l'idée d'intercaler un chariot intermédiaire entre un chariot supportant le lit, appelé support patient et le piédestal du dispositif. Ces deux chariots se déplacent simultanément et selon la verticale par rapport au piédestal du dispositif. Grâce à une chaîne, ou autre, dont les extrémités sont fixées au piédestal et au support patient et qui passe au dessus d'une poulie fixée sur le chariot intermédiaire, on soulève le support patient en soulevant le chariot intermédiaire. Ceci permet un déplacement, d'une part, vertical et, d'autre part, de grande amplitude car le déplacement du support patient est égal au double de celui du chariot intermédiaire. Il suffit, donc, de déplacer le chariot intermédiaire d'environ trente centimètres, ce qui est possible dans la pratique au moyen d'une vis, pour obtenir un déplacement de l'ordre de soixante centimètres du support patient afin que le lit se situe en position haute aux alentours de cent vingt centimètres. L'invention satisfait alors aux contraintes imposées.

EP-A-0 283 083 divulgue un lit d'examen en porte-à-faux et à déplacement vertical selon le préambule de la revendication 1.

Ainsi, l'invention a pour objet un lit d'examen réglable en hauteur, et en porte-à-faux, utilisé de manière mobile à des fins médicales pour une tomographie par scintigraphie, comportant un piédestal, un moteur, un support patient,
caractérisé en ce qu'il permet un déplacement de grande amplitude du support patient et selon la verticale en comportant:
- un chariot intermédiaire
- des moyens, actionnés par le moteur, pour pousser, selon la verticale, le chariot intermédiaire en le faisant glisser par rapport au piédestal
- un support patient se déplaçant, selon la verticale, le long du chariot intermédiaire
- une poulie, fixée sur le chariot intermédiaire, supportant une chaîne dont les extrémités sont fixées au piédestal et au support patient afin de pouvoir soulever le support patient lorsque les moyens pour pousser le chariot intermédiaire soulèvent ce chariot et par conséquent la poulie
- le piédestal et le support patient comportant des patins glissant le long de quatre rails verticaux fixés sur le chariot intermédiaire, deux du côté du piédestal et deux du côté du support patient, afin de permettre, d'une part, le déplacement vertical du chariot intermédiaire par rapport au piédestal et, d'autre part, celui du support patient par rapport au chariot intermédiaire
- les rails présentant une forme à section hexagonale de manière à ce que les patins coopèrent respectivement avec au moins quatre des côtés de la section hexagonale pour pouvoir soutenir le support patient et le chariot intermédiaire dans des directions perpendiculaires aux côtés de l'hexagone de façon à éviter le basculement du support patient.

L'invention sera mieux comprise à la lecture de la description qui suit et à l'examen des figures qui l'accompagnent. Celles-ci ne sont données qu'à titre indicatif et nullement limitatif de l'invention. Les figures montrent
- figure 1: une vue en perspective d'un lit, conforme à l'invention, en face d'un appareil d'examen;
- figures 2 et 3: des coupes du lit, conforme à l'invention, en vue de côté et vue de dessus;
- figure 4: un exemple de configuration des rails et des patins permettant, d'une part, le déplacement du chariot intermédiaire par rapport au piédestal et, d'autre part, celui du support patient par rapport au chariot intermédiaire pour convenir à la mise en oeuvre de l'invention.
- figure 5: un lit, conforme à l'invention, incliné sous l'effet du poids d'un patient allongé sur le support patient.

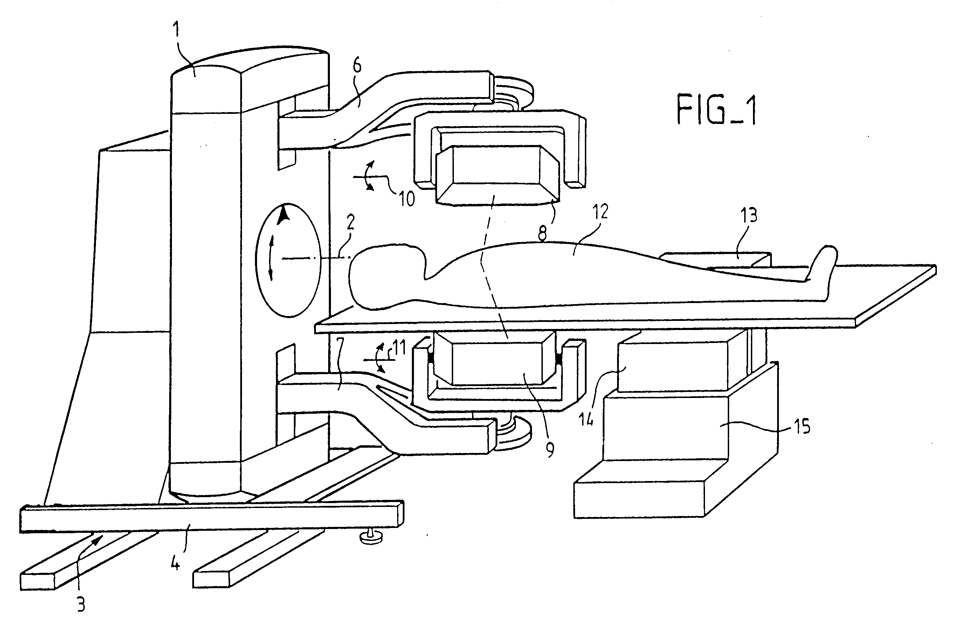

La figure 1 montre un patient 12 allongé sur un support patient 13, un chariot intermédiaire 14 et un piédestal 15 conformes à l'invention. Le support patient 13 est, dans un exemple, monté en porte à faux par rapport au piédestal 15. Le support patient a sensiblement la forme d'un L inversé. Le piédestal a la forme d'un L. On place un détecteur d'une gamma caméra en face du patient 12. Elle comporte une embase 1 tournante, susceptible de tourner autour d'un axe 2 de rotation. L'embase est maintenue en rotation sur un bâti 3 muni d'un socle 4. L'embase 1 comporte deux bras porteurs, respectivement 6 et 7, portant chacun une tête détectrice, respectivement 8 et 9, de la gamma caméra. Ces têtes détectrices peuvent pivoter autour d'axes 10 et 11 parallèles à l'axe 2. Ce mouvement est appelé, par la suite, angulation.

Lors d'un examen, on fixe l'angulation des têtes pour toute la durée de l'examen. On fait pivoter la gamma caméra et les têtes détectrices 8 et 9 autour du patient 12. Du fait que les têtes détectrices 8 et 9 ne sont pas horizontales, leur point de visée P dans le corps humain est décalé par rapport à l'axe 2 de rotation. Par conséquent, afin de viser toujours le même point P, on est obligé, lors de la rotation de la gamma caméra, d'animer la gamma caméra d'un mouvement de translation latérale tandis que le support patient 13 est animé d'un mouvement ascensionnel. Dans cette utilisation, le mouvement ascensionnel doit avoir une grande amplitude.

Le mouvement ascensionnel du support patient 13 s'effectue grâce, d'une part, au déplacement vertical du chariot intermédiaire 14 par rapport au piédestal 15 et, d'autre part, à celui du support patient 13 par rapport au chariot intermédiaire 14.

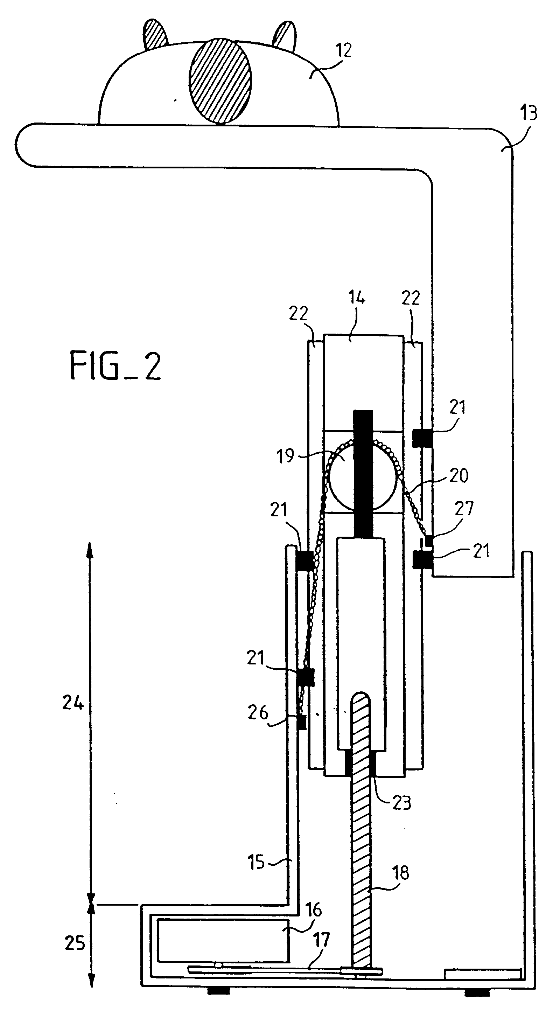

La figure 2 montre un moteur 16 permettant d'actionner, à l'aide d'une courroie 17, des moyens 18 de levage du chariot intermédiaire 14. Une poulie 19 est fixée sur le chariot intermédiaire 14. Une chaîne 20 passe au dessus de la poulie. Les extrémités 26 et 27 de la chaîne sont accrochées au piédestal 15 et au support patient 13. Le déplacement du chariot intermédiaire 14 et du support patient 13 s'effectuent grâce à des patins 21 qui glissent le long de rails 22.

Dans l'invention, on actionne les moyens 18 de levage pour soulever le chariot intermédiaire 14. On utilise, notamment, une vis 18 sans fin qui se visse dans un écrou 23 fixé à la partie inférieure du chariot intermédiaire 14. Le mouvement de rotation de la vis 18 est provoqué par le moteur 16 et est transmis à la vis 18 à l'aide de la courroie 17. On aurait pu aussi utiliser une vis engagée dans un écrou solidaire du piédestal 15. Celle-ci pousserait par en dessous le chariot intermédiaire 14 qui aurait été posé sur un patin au bout de la vis (ceci n'a pas été représenté sur les figures).

En soulevant le chariot intermédiaire 14, on soulève aussi la poulie 19. La poulie 19 entraîne, dans un mouvement vers le haut, l'extrémité 27 de la chaîne 20 fixée au support patient 13. Ainsi, lorsque le chariot intermédiaire 14 se déplace d'une certaine longueur, le support patient 13 se déplace du double. En effet, comme la chaîne 20 est maintenue au piédestal 15 par son extrémité 26, l'élévation du chariot 14 entraîne l'enroulement de la chaîne 20 sur la poulie 19.

Pour satisfaire aux contraintes imposées et citées plus haut, on utilise les dimensions suivantes. Celles-ci ne sont données qu'à titre indicatif et nullement limitatif de l'invention. La partie verticale du piédestal 15, représentée par la flèche 24, a pour longueur 45 centimètres, celle représentée par la base du piédestal, flèche 25, a pour longueur 10 centimètres. Ainsi, lorsque le support patient 13 et le chariot intermédiaire 14 sont complètement à l'intérieur du piédestal 15, on obtient une position basse de 55 centimètres (45+10). On utilise, de plus, des rails 22 d'une longueur de 55 centimètres. Mais, on limite le déplacement du chariot intermédiaire 14 par rapport au piédestal 15 et le déplacement du support patient 13 par rapport au chariot intermédiaire 14 à une longueur de 32 centimètres. En comptabilisant ces deux déplacements et la hauteur minimale, on permet au support patient 13 de s'élever à une hauteur maximale située à environ 120 centimètres (55 + 32 *2) au dessus du sol. En position maximale, chaque rail 22 porte alors au minimum sur 23 centimètres contre le piédestal 15 ou le support patient 13. Dans ce but, la hauteur du chariot intermédiaire 14 est de l'ordre de 55 cm lui aussi.

Les valeurs des contraintes de charge sont amplifiées à grande hauteur. Par exemple, on suppose que la masse du patient 12 et du support patient 13 ne peut excéder 200Kg. On considère que le support patient 13 est monté en porte à faux. On admet qu'un coefficient de sécurité normal est de l'ordre de six. Dans ces conditions, il faut prévoir que l'ensemble puisse soutenir jusqu'à 1.2 tonnes. Le fait que chaque rail 22 porte, en position haute, sur 23 centimètres, permet alors de résister a ces contraintes et de satisfaire aux conditions d'élévation verticale.

Concernant la chaîne 20, elle doit, de préférence, être de telle sorte qu'en position basse, la partie de la chaîne comprise entre la poulie 19 et l'attache 27 de cette chaîne 20 au support patient 13 soit d'une longueur sensiblement égale à 32 centimètres pour que le support patient 13 puisse se déplacer par rapport au chariot intermédiaire 14 de 32 centimètres. Cette condition impose que la poulie 19 soit fixée à plus de 32 centimètres au dessus de la partie inférieure du chariot intermédiaire 14 et que l'attache 27 au niveau du support patient 13 soit située à environ 32 centimètres en dessous de la poulie 19 quand cette dernière est en position basse. Cette condition, une fois réalisée, implique que la chaîne 20 ait une longueur nécessairement supérieure à 32 centimètres et permet, ainsi, au chariot intermédiaire 14 de se déplacer de 32 centimètres. L'autre extrémité 26 de la chaîne 20, celle fixée au piédestal 15, peut se situer à n'importe quelle hauteur sur le piédestal 15. De préférence, on attache la chaîne 20 le plus près de la position'basse de la poulie 19 pour gagner en longueur de chaîne.

La poulie 19 doit être fixée à au moins 32 centimètres au dessus de la partie inférieure du chariot intermédiaire 14. Comme on peut le remarquer sur la figure 2, à l'endroit où l'on fixe la poulie 19, il faut prévoir un passage de la chaîne au travers du chariot intermédiaire 14 tout en conservant la continuité des rails 22.

Dans un exemple, on fixe la poulie 19 à environ 32 centimètres au dessus de la partie inférieure du chariot intermédiaire 14. Ceci présente l'avantage que, placée à cette hauteur, elle joue le rôle de butée. En effet, comme l'attache 27 de la chaîne 20 au support patient 13 ne peut jamais se trouver à un niveau supérieur à celui de la poulie 19, le déplacement du support patient 13 par rapport au chariot intermédiaire 14 se trouve limité, par la position de la poulie 19, à 32 centimètres.

Pour ajuster ou modifier de quelques centimètres les positions maximale et minimale du support patient 13, on prévoit un réglage en hauteur de la poulie 19 et de l'attache 27 de la chaîne 20 au support patient 13. Pour cela, on utilise des rails (non représentés sur la figure), fixés respectivement au chariot intermédiaire 14 et au support patient 13, le long desquels on déplace, si nécessaire, la poulie 19 et l'attache 27.

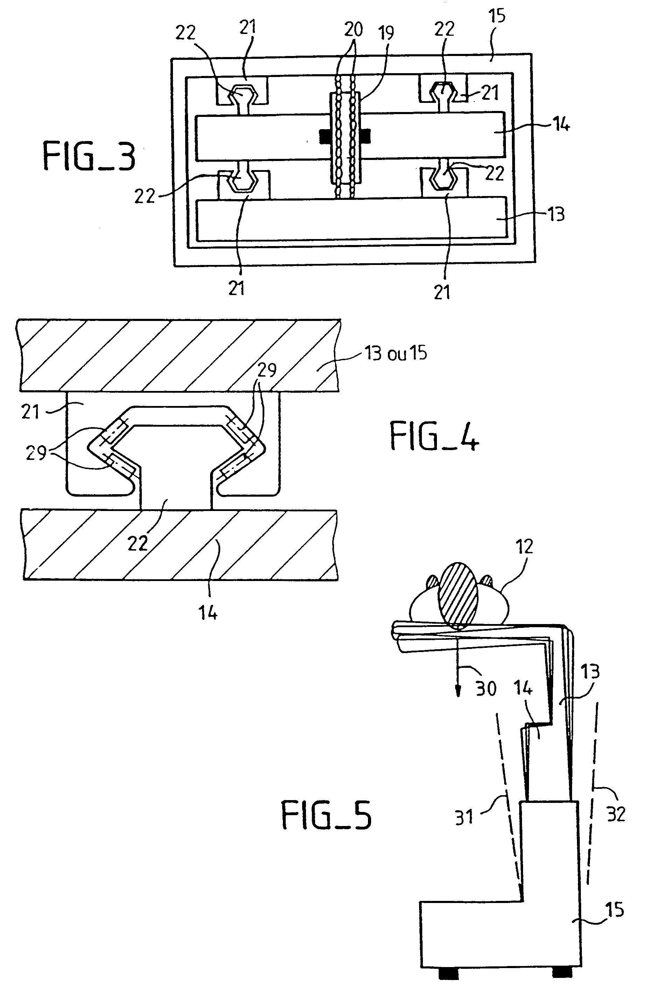

De plus, pour des raisons de sécurité, on utilise une chaîne 20 double, représentée à la figure 3, c'est à dire comportant deux enchaînements de mailles, placés l'un à côté de l'autre. On peut aussi utiliser deux poulies autour desquelles passent deux chaînes distinctes. Toujours pour les mêmes raisons, on exclut d'utiliser des courroies car elles sont plus fragiles que les chaînes.

La chaîne peut être remplacée par un autre dispositif. On peut, par exemple, concevoir que le piédestal 15 et le support patient 13 comportent des crémaillères en vis à vis. Une roue dentée s'engrène dans ces deux crémaillères à la fois. Elle tourne sur un axe fixé au chariot intermédiaire 14. Elle provoque l'élévation du support patient 13 par rapport au chariot intermédiaire 14 lorsque ce chariot intermédiaire 14 est poussé vers le haut. Ceci n'a pas été représenté sur les figures.

Comme le représente la figure 3 qui reprend en coupe les mêmes éléments, quatre rails 22 sont fixés au chariot intermédiaire 14 (deux du côté du piédestal 15 et deux du côté du support patient 13). Ils sont notamment du type THK. Sur cette figure, on remarque aussi quatre patins 21. Dans la réalisation pratique, les patins sont au nombre de huit. Les quatre autres patins ne sont pas représentés car ils sont situés à un niveau inférieur au plan de la coupe de la figure 3. Les patins 21 sont fixés pour une moitié sur le support patient 13 et pour l'autre sur le piédestal 15. On pourrait aussi fixer les patins 21 sur le chariot intermédiaire 14 et les rails 22 sur le piédestal 15 et le support patient 13. C'est équivalent. Pour pouvoir être utiles quand le support patient 13 est en position haute, les quatre patins 21 du piédestal 15 sont situés en haut du piédestal 15 alors que ceux du support patient 13 sont situés en bas du support patient 13. Dans un exemple, les patins 21 du support patient 13 ainsi que ceux du piédestal 15, sont espacés, l'un de l'autre, de 15 centimètres en hauteur et de 25 centimètres en largeur.

La figure 4 montre que les rails 22 de type THK présentent des profils ayant une forme hexagonale. Ainsi, ils permettent aux patins 21 d'exercer des efforts dans les trois directions perpendiculaires aux côtés de l'hexagone. Les efforts évitent le basculement du support patient 13. Ceci est un avantage par rapport à des glissières de type rectangulaire qui ne travaillent que dans deux directions.

Les patins 21 comportent des roulements 29 amovibles et interchangeables. Si on place des roulements 29 plus gros, on réduit le jeu entre les rails 22 et les patins 21. De ce fait, on consolide davantage l'ensemble, c'est à dire le chariot intermédiaire 14, le support patient 13 et le piédestal 15. Le choix de roulements 29 plus gros doit être fait en fonction de la puissance disponible au moteur 16. Les roulements 29 plus gros exercent une résistance plus importante. L'avantage des rails 22 de type THK est donc bien mis à profit ici. Avec des roulements 29 plus gros, on a une meilleure rigidité de l'ensemble tout en évitant d'avoir à plaquer le chariot intermédiaire 14 contre le piédestal 15 et le support patient 13 contre le chariot intermédiaire 14. Avec le profil hexagonal des rails 22 de type THK, deux faces des patins 21 exercent des actions en réaction l'une de l'autre sur un même rail.

Comme il l'a été indiqué plus haut, le support patient 13 est, en général, monté en porte à faux par rapport au piédestal 15. Donc, sous l'effet du poids du patient 12 représenté sur la figure 5 par la flèche 30, le chariot intermédiaire 14 et le support patient 13 tendent à s'incliner suivant une direction 31 (en pointillé) décalée par rapport à la verticale. Pour remédier à ce problème, le chariot intermédiaire 14 et le support patient 13 sont conçus pour s'élever, quand il n'y a pas de patient 12 sur le support patient 13, suivant une direction 32 (en pointillé), légèrement décalée en biais de l'autre côté par rapport à la verticale. Ils s'élèvent verticalement si un patient 12 est allongé sur le support patient 13, sous l'effet du poids 30 de ce patient. Cette élévation est sensiblement une verticale pour tous les niveaux d'élévation.

## Revendications

1. Lit d'examen réglable en hauteur, et en porte-à-faux, utilisé de manière mobile à des fins médicales pour une tomographie par scintigraphie, comportant-un piédestal (15), un moteur (16), un support patient (13),
caractérisé en ce qu'il permet un déplacement de grande amplitude du support patient (13) et selon la verticale en comportant:
- un chariot intermédiaire (14)
- des moyens (18), actionnés par le moteur (16), pour pousser, selon la verticale, le chariot intermédiaire (14) en le faisant glisser par rapport au piédestal (15)
- un support patient (13) se déplaçant, selon la verticale, le long du chariot intermédiaire (14)
- une poulie (19), fixée sur le chariot intermédiaire (14), supportant une chaîne (20) dont les extrémités (26) et (27) sont fixées au piédestal (15) et au support patient (13) afin de pouvoir soulever le support patient (13) lorsque les moyens (18) pour pousser le chariot intermédiaire (14) soulèvent ce chariot (14) et par conséquent la poulie (19)
- le piédestal (15) et le support patient (13) comportant des patins (21) glissant le long de quatre rails verticaux (22) fixés sur le chariot intermédiaire (14), deux du côté du piédestal (15) et deux du côté du support patient (13), afin de permettre, d'une part, le déplacement vertical du chariot intermédiaire (14) par rapport au piédestal (15) et, d'autre part, celui du support patient (13) par rapport au chariot intermédiaire (14)
- les rails (22) présentant une forme à section hexagonale de manière à ce que les patins (21) coopèrent respectivement avec au moins quatre des côtés de la section hexagonale pour pouvoir soutenir le support patient (13) et le chariot intermédiaire (14) dans des directions perpendiculaires aux côtés de l'hexagone de façon à éviter le basculement du support patient (13).

2. Lit selon la revendication 1, caractérisé en ce que les moyens (18) pour pousser le chariot intermédiaire (14) comportent une vis (18) qui soulève le chariot intermédiaire (14).

3. Lit selon l'une quelconque des revendications 1 à 2, caractérisé en ce que les moyens (18) pour pousser le chariot intermédiaire (14) comportent une vis (18) sans fin qui soulève le chariot intermédiaire (14) en se vissant dans un écrou (23) fixé à la partie inférieure du chariot intermédiaire (14).

4. Lit selon l'une quelconque des revendications 1 à 3, caractérisé en ce que les patins (21) comportent des roulements (29) amovibles et interchangeables afin de permettre de réduire le jeu entre les rails (22) et les patins (21).

5. Lit selon l'une quelconque des revendications 1 à 4, caractérisé en ce que la poulie (19) et le point d'attache (27) de la chaîne (20) sur le support patient (13) sont réglables en hauteur.

6. Lit selon l'une quelconque des revendications 1 à 5, caractérisé en ce que l'attache (26) de la chaîne (20) au piédestal (15) se situe à une position haute, prés de la poulie (19), pour pouvoir raccourcir la longueur de la chaîne (20).

7. Lit selon l'une quelconque des revendications 1 à 6, caractérisé en ce que le support patient (13) est monté en porte à faux par rapport au piédestal (15).

8. Lit selon l'une quelconque des revendications 1 à 7, caractérisé en ce que le chariot intermédiaire (14) et le support patient (13) sont orientés pour s'élever, quand il n'y a pas de patient (12) sur le support patient (13), suivant un axe (32), légèrement décalé en biais par rapport à la verticale, afin qu'ils s'élèvent verticalement, si un patient (12) est allongé dessus, sous l'effet de son poids (30).

## Claims

1. An examination couch adjustable for height and cantilever, used in a mobile manner for medical purposes for scinitgraphic tomography, comprising a pedestal (15), motor (16), patient support (13),
characterised by the fact that it permits movement of great amplitude of the patient support (13) and vertically by comprising:
- an intermediate carriage (14)
- means (18), activated by the motor (16), for moving the intermediate carriage (14) vertically by causing it slide in relation to the pedestal (15)
- a patient support (13) moving vertically along the intermediate carriage (14)
- a pulley (19), fixed to the intermediate carriage (14), supporting a chain (20) the ends (26) and (27) of which are fixed to the pedestal (15) and to the patient support (13) to enable the patient support (13) to be raised when the means (18) for moving the intermediate carriage (14) raises this carriage (14) and consequently the pulley (19)
- the pedestal (15) and the patient support (13) comprising runners (21) sliding along four vertical rails (22) fixed to the intermediate carriage (14), two on the pedestal (15) side and two on the patient support (13) side, in order to permit, firstly, the vertical movement of the intermediate carriage (14) in relation to the pedestal (15) and, secondly, that of the patient support (13) in relation to the intermediate carriage (14)
- rails (22) with a hexagonal cross section such that the runners (21) cooperate respectively with at least four sides of the hexagonal cross section to enable the patient support (13) and the intermediate carriage (14) to be raised in directions perpendicular to the sides of the hexagon so as to prevent the patient support (13) from tipping over.

2. A couch according to claim 1, characterised by the fact that the means (18) for moving the intermediate carriage (14) comprises a screw (18) which raises the intermediate carriage (14).

3. A couch according to either of claims 1 to 2, characterised by the fact that the means (18) for moving the intermediate carriage (14) comprises a worm screw (18) which raises the intermediate carriage (14) by turning in a nut (23) fixed to the lower part of the intermediate carriage (14).

4. A couch according to any of claims 1 to 3, characterised by the fact that the runners (21) comprise removable, interchangeable bearings (29) to reduce the clearance between the rails (22) and the runners (21).

5. A couch according to any of claims 1 to 4, characterised by the fact that the pulley (19) and the point of attachment (27) of the chain (20) to the patient support (13) are adjustable in height.

6. A couch according to any of claims 1 to 5, characterised by the fact that the attachment (26) of the chain (20) to the pedestal (15) is situated at a high position, near to the pulley (19), to shorten the length of the chain (20).

7. A couch according to any of claims 1 to 6, characterised by the fact that the patient support (13) is cantilever mounted in relation to the pedestal (15).

8. A couch according to any of claims 1 to 7, characterised by the fact that the intermediate carriage (14) and the patient support (13) are orientated so as to be raised, when there is no patient (12) on the patient support (13), on an axis (32) set at a slight angle to the vertical, so that they rise vertically when a patient (12) is lying on top, under the effect of his/her weight (30).

## Patentansprüche

1. Höhenverstellbare und überstehende Untersuchungsliege, die beweglich zu medizinischen Zwecken zu einer Szintigraphie-Tomographie verwendet wird und einen Sockel (15), einen Motor (16), eine Patientenauflage (13) umfaßt,
dadurch gekennzeichnet, daß sie eine Verschiebung der Patientenauflage (13) mit großem Ausschlag gemäß der Vertikalen erlaubt und umfaßt:
- einen Zwischenwagen (14)
- von dem Motor (16) betätigte Vorrichtungen (18) zum Schieben des Zwischenwagens (14) entlang der Vertikalen, indem man ihn bezüglich dem Sockel (15) gleiten läßt
- eine Patientenauflage (13), die sich gemäß der Vertikalen entlang des Zwischenwagens (14) bewegt
- eine Scheibe (19), die an dem Zwischenwagen (14) angebracht ist und eine Kette (20) tragt, deren Enden (26) und (27) an dem Sockel (15) und an der Patientenauflage (13) befestigt sind, um die Patientenauflage (13) anzuheben, wenn die Vorrichtungen (18) zum Schieben des Zwischenwagens (14) diesen Wagen (14) und somit die Scheibe (19) anheben
- den Sockel (15) und die Patientenauflage (13), die Gleitschuhe (21) aufweist, die entlang vier vertikaler Schienen (22) gleiten, die an dem Zwischenwagen (14) befestigt sind, und zwar zwei auf der Seite des Sockels (15) und zwei auf der Seite der Patientenauflage (13), um einerseits die vertikale Verschiebung des Zwischenwagens (14) bezüglich dem Sockel (15) und andererseits die der Patientenauflage (13) bezüglich dem Zwischenwagen (14) zu erlauben
- wobei die Schienen (22) eine Form mit sechseckigem Querschnitt haben, damit die Gleitschuhe (21) jeweils mit mindestens vier der Seiten des sechseckigen Querschnitts zusammenwirken, um die Patientenauflage (13) und den Zwischenwagen (14) in zu den Seiten des Sechsecks quer verlaufenden Richtungen halten zu können, um ein Kippen der Patientenauflage (13) zu verhindern.

2. Liege nach Anspruch 1, dadurch gekennzeichnet, daß die Vorrichtungen (18) zum Schieben des Zwischenwagens (14) eine Schraube (18) umfassen, die den Zwischenwagen (14) anhebt.

3. Liege nach einem der Ansprüche 1 bis 2, dadurch gekennzeichnet, daß die Vorrichtungen (18) zum Schieben des Zwischenwagens (14) eine Schraube (18) ohne Ende umfassen, die den Zwischenwagen (14) anhebt, indem sie in eine Mutter (23) geschraubt wird, die im unteren Bereich des Zwischenwagens (14) angebracht ist.

4. Liege nach einem der Ansprüche 1 bis 3, dadurch gekennzeichnet, daß die Gleitschuhe (21) wegnehmbare und untereinander austauschbare Lager (29) umfassen, um eine Verringerung des Spiels zwischen den Schienen (22) und den Gleitschuhen (21) zu ermöglichen.

5. Liege nach einem der Ansprüche 1 bis 4, dadurch gekennzeichnet, daß die Scheibe (19) und der Befestigungspunkt (27) der Kette an der Patientenauflage (13) höhenverstellbar sind.

6. Liege nach einem der Ansprüche 1 bis 5, dadurch gekennzeichnet, daß die Befestigung (26) der Kette (20) am Sockel (15) in einer hohen Position nahe der Scheibe (19) angeordnet ist, damit die Länge der Kette (20) verkürzt werden kann.

7. Liege nach einem der Ansprüche 1 bis 6, dadurch gekennzeichnet, daß die Patientenauflage (13) vorstehend bezüglich dem Sockel (15) montiert ist.

8. Liege nach einem der Ansprüche 1 bis 7, dadurch gekennzeichnet, daß der Zwischenwagen (14) und die Patientenauflage (13) so ausgerichtet sind, daß sie sich, wenn kein Patient auf der Patientenauflage (13) liegt, entlang einer Achse (32) erheben, die leicht schräg bezüglich der Vertikalen versetzt ist, damit sie sich, wenn ein Patient (12) darauf liegt, unter seinem Gewicht (30) vertikal erheben.
